Europäisches Patentamt

European Patent Office

Office européen des brevets

(19)

(11) Veröffentlichungsnummer: **0 247 456**
**B1**

(12) **EUROPÄISCHE PATENTSCHRIFT**

(45) Veröffentlichungstag der Patentschrift: **18.04.90**

(51) Int. Cl.⁵: **C 07 C 209/84, C 07 C 211/58**

(21) Anmeldenummer: **87107041.3**

(22) Anmeldetag: **15.05.87**

(54) **Verfahren zur Reinigung von 1-Amino-5-hydroxynaphthalin enthaltendem 1,5-Diaminonaphthalin.**

(30) Priorität: **27.05.86 DE 3617838**

(43) Veröffentlichungstag der Anmeldung:
**02.12.87 Patentblatt 87/49**

(45) Bekanntmachung des Hinweises auf die
Patenterteilung:
**18.04.90 Patentblatt 90/16**

(84) Benannte Vertragsstaaten:
**CH DE FR GB IT LI**

(56) Entgegenhaltungen:
**DE-A-1 643 377**
**DE-A-1 768 743**
**DE-A-2 824 248**
**US-A-4 067 903**

**PATENT ABSTRACTS OF JAPAN, Band 4, Nr. 46
(C-6)528r; & JP - A - 55 19211**

**CHEMICAL ABSTRACTS, Band 83, Nr. 11, 15.
September 1975, Zusammenfassungsnr. 96853t,
K. WATANABE et al.: "Purification of 1,5-
diaminonaphthalene"; & JP - A - 50 49264**

(73) Patentinhaber: **BAYER AG
D-5090 Leverkusen 1 Bayerwerk (DE)**

(72) Erfinder: **Mayer, Dietmar, Dr.
In den Wiesen 1
D-5060 Bergisch Gladbach 2 (DE)**
Erfinder: **Wahle, Karl-Heinz
Kolberger Strasse 93
D-5090 Leverkusen 3 (DE)**

Courier Press, Leamington Spa, England.

# Beschreibung

Die vorliegende Erfindung betrifft ein Verfahren zur Reinigung von 1-Amino-5-hydroxynaphthalin enthaltendem 1,5-Diaminonaphthalin.

Es ist bekannt, daß Amine, beispielsweise 1,5-Diamino-naphthalin, von Verbindungen mit phenolischen Gruppen, wie 1-Amino-5-hydroxynaphthalin (Purpurol), durch Zusatz von Basen infolge eintretender Löslichkeitsunterschiede durch Behandlung mit wäßrigen und organischen Lösungsmitteln voneinander getrennt werden können (vgl. z.B. H. Staudinger/W. Kern: Anleitung zur organischen qualitativen Analyse, Springer-Verlag 1955, S. 95 und 155). Sollen allerdings zugleich auch nichtflüchtige Verunreinigungen bei der Reinigung des 1,5-Diaminonaphthalins entfernt werden, so stellt das bekannte Verfahren bei der Durchführung im technischen Maßstab einen nicht unerheblichen Mehraufwand dar, da vor der eigentlichen Endreinigung durch Sublimation zusätzlichen Arbeitsschritte anfallen. Solche zusätzlichen Arbeitsschritte sind beispielsweise das Behandeln des Rohproduktes mit Laugen bei erhöhter Temperatur in wäßriger Suspension sowie die Isolierung des in wäßrigem Medium unlöslichen 1,5-Diaminonaphthalins von der wäßrigen, das Purpurol in Form des Natrium-Salzes enthaltenden Lösung durch Filtration.

Es wurde nun ein Verfahren zur Reinigung von 1-Amino-5-hydroxynaphthalin enthaltendem 1,5-Diaminonaphthalin gefunden, das dadurch gekennzeichnet ist, daß man das zu reinigende 1,5-Diaminonaphthalin in Gegenwart von basischen anorganischen Verbindungen im Vakuum sublimiert.

Das erfindungsgemäß zu reinigende 1,5-Diaminonaphthalin, das durch Amidierung von 1,5-Dihydroxynaphthalin (Bucherer-Reaktion) hergestellt wurde, enthält neben anderen Verunreinigungen insbesondere das bei der Weiterverarbeitung besonders störende 1-Amino-5-hydroxynaphthalin in einer Menge von etwa 1—1,5 Gew.-%. Selbstverständlich ist es auch nach dem erfindungsgemäßen Verfahren möglich, 1,5-Diaminonaphthalin zu reinigen, das einen höheren Gehalt an 1-Amino-5-hydroxynaphthalin enthält.

Die Reinigung des rohen 1,5-Diaminonaphthalins geschieht erfindungsgemäß durch Sublimation in Gegenwart von basischen anorganischen Verbindungen im Vakuum. Als basische anorganische Verbindungen kommen insbesondere die Alkalihydroxide infrage, wie Natriumhydroxid, Kaliumhydroxid und/oder Lithiumhydroxid. Besonders vorteilhaft wird die Sublimation in Gegenwart von Natriumhydroxid durchgeführt.

Die basischen anorganischen Verbindungen werden bei dem erfindungsgemäßen Verfahren in einer Menge von etwa 1—5 Gew.-%, bevorzugt 1,5—2,5 Gew., bezogen auf das eingesetzte 1,5-Diaminonaphthalin, zugesetzt.

Die Sublimation wird bei Temperaturen von etwa 180—250°C, bevorzugt bei 200—230°C, durchgeführt. Der Druck beträgt dabei etwa 0,5—30 mbar, bevorzugt 0,6—1,5 mbar.

Beispielsweise kann das erfindungsgemäße Verfahren so durchgeführt werden, daß man das rohe 1,5-Diaminonaphthalin in trockner Form mit pulverisiertem Alkalihydroxid vermischt und anschließend einer Vakuumsublimation unterwirft. Es ist jedoch auch möglich, das rohe, trockne oder rohe, feuchte 1,5-Diaminonaphthalin mit einer wäßrigen Lösung der genannten Alkalihydroxiden zu vermischen und nach der Trocknung einer Vakuumsublimation zu unterwerfen. Dabei ist darauf zu achten, daß die basischen anorganischen Verbindungen in einer Menge zugegeben werden, die zur Bindung des Purpurols als Alkalisalz ausreicht. Darüber hinaus ist es möglich, das rohe, feuchte 1,5-Diaminonaphthalin im Rahmen der Isolierung der Feuchtware aus dem Produktionsprozeß mit Lösungen der vorgenannten anorganischen Basen in ausreichender Menge zu waschen, zu trocknen und anschließend im Vakuum zu sublimieren.

Das erfindungsgemäße Verfahren wird im allgemeinen wie folgt durchgeführt:

Das rohe 1,5-Diaminonaphthalin, das durch Aminierung von 1,5-Dihydroxynaphthalin erhalten wurde und einen Purpurol-Anteil von etwa 1—1,5 Gew.-% besitzt, wird mit geeigneten anorganischen Basen, wie Natriumhydroxid, vermischt. Dabei werden die anorganischen Basen in einer Menge zugesetzt, die ausreicht, um das gesamte Purpurol in die nichtflüchtige Salzform überzuführen. Der Basenzusatz erfolgt zweckmäßigerweise im Rahmen von ohnehin bei der Isolierung und Vorbehandlung der Rohware erforderlichen Arbeitsschritten, so z.B. bei der Isolierung mittels Filtration durch Waschen mit wäßrigen Lösungen der vorgenannten anorganischen Basen oder bei der Trocknung durch Zusatz der anorganischen Basen in fester oder gelöster Form vor dem Trocknungsvorgang. Bei der anschließenden Reinigung durch Vakuumsublimation verbleibt das Purpurol als nichtflüchtiges Salz im Sublimationsrückstand und kann so abgetrennt werden.

Das erfindungsgemäße Verfahren ermöglicht in einfacher Weise ohne technische Änderungen bestehender Anlagen die Herstellung eines reinen 1,5-Diaminonaphthalins, das die bei der Weiterverarbeitung störende Nebenkomponente Purpurol praktisch nicht mehr enthält.

Obwohl unter den drastischen Reaktionsbedingungen (hohe Temperaturen, Zusatz von stark basisch reagierenden Verbindungen) mit einer Zersetzung der Produkte gerechnet werden mußte, wird die Sublimationsausbeute bei dem erfindungsgemäßen Verfahren überraschenderweise nicht beeinträchtigt. Sie liegt bei etwa 94—96%.

## Beispiel 1

142,5 g 1,5-Diaminonaphthalin (roh/trocken) mit einem Purpurolgehalt von 1 Gew.-% wurden mit 7,5 g Ätznatron und 100 ml Wasser verrührt und die Mischung anschließend in Vakuum bei 75°C getrocknet. Nach Sublimation im Vakuum bei 220°C/0,6 mbar erhielt man 95% vom Einsatzprodukt (v.E.) an 1,5-Diaminonaphthalin (subl.), in dem Purpurol mittels Dünnschichtchromatographie (DC) nicht mehr nachweisbar war.

### Beispiel 2

150 g 1,5-Diaminonaphthalin (roh/feucht) mit einem Purpurolgehalt von 1—1,5 Gew.-% wurden mit 3 g 50%iger Natronlauge und 20 ml Wasser vermischt und die Mischung im Vakuum bei 75°C getrocknet. Nach Sublimation im Vakuum bei 220°C/0,7—0,8 mbar erhielt man 95,5% v.E. an 1,5-Diaminonaphthalin (subl.). Purpurol war mittels DC im Sublimat nicht mehr nachweisbar.

### Beispiel 3

150 g 1,5-Diaminonaphthalin (roh/feucht) mit einem Purpurolgehalt von 1,5 Gew.-% wurden unter Zusatz von 1,56 g Ätznatron in Form von handelsüblichen Perlen von 2—3 mm Durchmesser im Rotationsverdampfer bei 75°C im Vakuum der Wasserstrahlpumpe getrocknet und anschließend einer Vakuumsublimation bei 220°C/0,7 mbar unterworfen. Man erhielt 94,8% v.E. an 1,5-Diaminonaphthalin (subl.), in dem Purpurol mittels DC nicht mehr nachweisbar war.

### Beispiel 4

150 g 1,5-Diaminonaphthalin (roh/feucht) mit einem Purpurolgehalt von 1,5 Gew.-% wurden unter Zusatz von 6 g Kalilauge (50%ig) im Rotationsverdampfer bei 75°C im Vakuum getrocknet und anschließend einer Vakuumsublimation bei 220°C/0,4 mbar unterworfen. Man erhielt 91,8% v.E. an 1,5-Diaminonaphthalin (subl.), in dem Purpurol mittels Dünnschichtchromatographie nicht mehr nachweisbar war.

### Beispiel 5

50 g 1,5-Diaminonaphthalin (subl.) mit einem Purpurolgehalt von 1 Gew.-% wurden mit 2 g Lithiumhydroxid (pulv.) gemischt und anschließend einer Vakuumsublimation bei 220°C/0,8 mbar unterworfen. Man erhielt 94,5% v.E. an 1,5-Diaminonaphthalin-Sublimat, in dem Purpurol mittels DC nicht mehr nachweisbar war.

### Patentansprüche

1. Verfahren zur Reinigung von 1-Amino-5-hydroxynaphthalin enthaltendem 1,5-Diaminonaphthalin, dadurch gekennzeichnet, daß man das zu reinigende 1,5-Diaminonaphthalin in Gegenwart von basischen anorganischen Verbindungen unter vermindertem Druck sublimiert.

2. Verfahren nach Anspruch 1, dadurch gekennzeichnet, daß man als basische anorganische Verbindungen Alkalihydroxide zusetzt.

3. Verfahren nach Ansprüchen 1 und 2, dadurch gekennzeichnet, daß man als Alkalihydroxide Natriumhydroxid, Kaliumhydroxid und/oder Lithiumhydroxid zusetzt.

4. Verfahren nach Ansprüchen 1 bis 3, dadurch gekennzeichnet, daß man die Sublimation bei Temperaturen von 180—250°C durchführt.

5. Verfahren nach Ansprüchen 1 bis 4, dadurch gekennzeichnet, daß man die Sublimation bei einem Druck von 0,5—30 mbar durchführt.

6. Verfahren nach Ansprüchen 1 bis 5, dadurch gekennzeichnet, daß man die anorganischen basischen Verbindungen in einer Menge von 1—5 Gew.-%, bezogen auf das eingesetzte 1,5-Diaminonaphthalin, zusetzt.

### Revendications

1. Procédé d'épuration de 1,5-diaminonaphtalène contenant du 1-amino-5-hydroxynaphtalène, caractérisée en ce qu'on sublime sous pression réduite le 1,5-diamino-naphtalène à épurer en présence de composés basiques inorganiques.

2. Procédé suivant la revendication 1, caractérisé en ce qu'on ajoute des hydroxydes alcalins comme composés basiques inorganiques.

3. Procédé suivant les revendications 1 et 2, caractérisé en ce qu'on ajoute comme hydroxydes alcalins de l'hydroxyde de sodium, de l'hydroxyde de potassium et/ou de l'hydroxyde de lithium.

4. Procédé suivant les revendications 1 à 3, caractérisé en ce qu'on conduit la sublimation à des températures de 180 à 250°C.

5. Procédé suivant les revendications 1 à 4, caractérisé en ce qu'on conduit la sublimation à une pression de 0,5 à 30 mbars.

6. Procédé suivant les revendications 1 à 5, caractérisé en ce qu'on ajoute les composés basiques inorganiques en une quantité de 1 à 5% en poids par rapport au 1,5-diaminonaphtalène utilisé.

### Claims

1. Process for the purification of 1,5-diaminonaphthalene which contains 1-amino-5-hydroxynaphthalene, characterized in that the 1,5-diaminonaphthalene to be purified is sublimed under reduced pressure in the presence of basic inorganic compounds.

2. Process according to Claim 1, characterized in that alkali metal hydroxides are added as basic inorganic compounds.

3. Process according to Claims 1 and 2, characterized in that sodium hydroxide, potassium hydroxide and/or lithium hydroxide are added as alkali metal hydroxides.

4. Process according to Claims 1 to 3, characterized in that the sublimation is carried out at temperatures of 180—250°C.

5. Process according to Claims 1 to 4, characterized in that the sublimation is carried out at a pressure of 0.5—30 mbar.

6. Process according to Claims 1 to 5, characterized in that the inorganic basic compounds are added in an amount of 1—5% by weight, relative to the 1,5-diaminonaphthalene employed.